# EUROPÄISCHE PATENTSCHRIFT

(11) **EP 1 438 294 B1**
(45) Veröffentlichungstag und Bekanntmachung des Hinweises auf die Patenterteilung: **05.12.2007**
(21) Anmeldenummer: 02802296.0
(22) Anmeldetag: 17.10.2002
(51) Int. Cl.: C07D 233/54, C07D 233/64, A61Q 5/10

(54) **ENTWICKLERKOMPONENTEN UND DEREN VERWENDUNG ZUR FÄRBUNG KERATINISCHER FASERN**
DEVELOPER CONSTITUENTS AND THEIR USE FOR DYING KERATIN FIBERS
COMPOSANTS DEVELOPPEURS ET UTILISATION POUR LA COLORATION DE FIBRES KERATINIQUES

(30) Priorität: 26.10.2001 DE 10152941
(43) Veröffentlichungstag der Anmeldung: 21.07.2004
(73) Patentinhaber: Henkel Kommanditgesellschaft auf Aktien, 40589 Düsseldorf (DE)
(72) Erfinder: KNÜBEL, Georg, 40219 Düsseldorf (DE); HÖFFKES, Horst, 40595 Düsseldorf (DE); MEINIGKE, Bernd, 51381 Leverkusen (DE); GIESA, Helmut, 40670 Meerbusch (DE)
(86) Internationale Anmeldenummer: PCT/EP2002/011622
(87) Internationale Veröffentlichungsnummer: WO 2003/037875

(56) Entgegenhaltungen:
- EP-A- 0 984 007
- EP-A- 1 116 711
- WO-A-99/03836

## Beschreibung

Die vorliegende Erfindung betrifft Mittel zum Färben keratinischer Fasern, die ein mit einer Imidazolylgruppe substituiertes p-Phenylendiaminderivat enthalten, die Verwendung dieser Verbindungen zur Färbung keratinischer Fasern, ein Verfahren zur Färbung von Fasern mit diesen Verbindungen sowie einige dieser p-Phenylendiaminderivate an sich.

Für das Färben von Keratinfasern, insbesondere menschlichen Haaren, spielen die sogenannten Oxidationsfärbemittel wegen ihrer intensiven Farben und guten Echtheitseigenschaften eine bevorzugte Rolle. Solche Färbemittel enthalten Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehrerer Kupplerkomponenten die eigentlichen Farbstoffe aus.

Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis(2'-hydroxyethyl)-p-phenylendiamin, 2-(2',5'-Diaminophenyl)-ethanol, 2-(2',5'-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxy-4,5,6-triaminopyrimidin, 2,4-Dihydroxy-5,6-diaminopyrimidin, 2,5,6-Triamino-4-hydroxypyrimidin und 1,3-N,N'-Bis(2'-hydroxyethyl)-N,N'-bis(4'-aminophenyl)-diamino-propan-2-ol.

Als Kupplerkomponenten werden in der Regel m-Phenylendiaminderivate, Naphthole, Resorcin und Resorcinderivate, Pyrazolone und m-Aminophenole verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin und 2-Methyl-4-chlor-5-aminophenol.

Gute Oxidationsfarbstoffvorprodukte sollen in erster Linie folgende Voraussetzungen erfüllen: Sie müssen bei der oxidativen Kupplung die gewünschten Farbnuancen in ausreichender Intensität und Echtheit ausbilden. Sie müssen ferner ein gutes Aufziehvermögen auf die Faser besitzen, wobei insbesondere bei menschlichen Haaren keine merklichen Unterschiede zwischen strapaziertem und frisch nachgewachsenem Haar bestehen dürfen (Egalisiervermögen). Sie sollen beständig sein gegen Licht, Wärme, Schweiß, Reibung und den Einfluss chemischer Reduktionsmittel, z.B. Dauerwellenflüssigkeiten. Schließlich sollen sie - falls als Haarfärbemittel zur Anwendung kommend - die Kopfhaut nicht zu sehr anfärben, und vor allem sollen sie in toxikologischer und dermatologischer Hinsicht unbedenklich sein. Weiterhin soll die erzielte Färbung durch Blondierung leicht wieder aus dem Haar entfernt werden können, falls sie doch nicht den individuellen Wünschen der einzelnen Person entspricht und rückgängig gemacht werden soll.

Allein mit einer Entwicklerkomponente oder einer speziellen Kuppler/Entwicklerkombination gelingt es in der Regel nicht, eine auf dem Haar natürlich wirkende Farbnuance zu erhalten. In der Praxis werden daher üblicherweise Kombinationen verschiedener Entwickler- und/oder Kupplerkomponenten eingesetzt. Es besteht daher ständig Bedarf an neuen, verbesserten Farbstoffkomponenten, die auch in toxikologischer und dermatologischer Hinsicht unproblematisch sind.

Im Rahmen der WO99/03836 werden oxidative Färbemittel beschrieben, die spezielle kationische Entwicklerkomponenten enthalten, die eine Imidazoliumgruppe enthalten können.

In der EP-A2-1 116 711 werden oxidative Färbemittel offenbart, die 2-Aminoalkyl-1n4-diaminobenzolderivate als Entwicklerkomponente enthalten, die eine Imidazolgruppe enthalten können.

Aufgabe der vorliegenden Erfindung war es daher, neue Entwicklerkomponenten zu entwickeln, die die an Oxidationsfarbstoffvorprodukte gestellten Anforderungen erfüllen und Färbungen in einem breiten Farbspektrum mit guten Echtheitseigenschaften ermöglichen.

Überraschenderweise wurde nun gefunden, dass spezielle Imidazol-substituierte p-Phenylendiaminderivate den an Oxidationsfarbstoffvorprodukte gestellten Anforderungen in einem hohen Maße genügen. Die erfindungsgemäßen Entwicklerkomponenten zeichnen sich durch intensive Farbergebnisse mit ausgezeichneter Schweißechtheit sowie Kaltwellechtheit aus. Der Farbausfall von Färbungen in Kombination mit herkömmlichen Farbstoffvorprodukten ist gegenüber den Ausfärbungen mit p-Toluylendiamin etwas in Bläuliche verschoben.

Ein erster Gegenstand der vorliegenden Erfindung sind daher Mittel zum Färben keratinischer Fasern, insbesondere menschlicher Haare, die in einem kosmetisch akzeptablen Träger mindestens ein p-Phenylendiaminderivat der Formel (I) 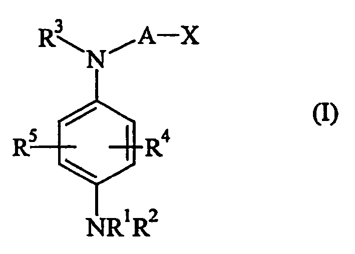 enthalten, wobei
- A steht für eine verzweigte oder unverzweigte Alkylengruppe mit 1 bis 6 Kohlenstoffatomen, die gegebenenfalls einen oder mehrere Substituenten, ausgewählt aus Hydroxygruppe(n) und Halogenatom(en), tragen kann,
- X steht für einen gegebenenfalls substituierten Imidazolylrest,
- R¹, R² und R³ stehen unabhängig voneinander für ein Wasserstoffatom, eine C₁- bis C₄-Alkylgruppe, eine C₁- bis C₄-Monohydroxyalkylgruppe oder eine C₂- bis C₆-Polyhydroxyalkylgruppe, und
- R⁴ und R⁵ stehen unabhängig voneinander für ein Wasserstoffatom, eine C₁- bis C₄-Alkylgruppe, eine C₁- bis C₄-Alkoxygruppe, eine C₁- bis C₄-Monohydroxyalkylgruppe, eine C₂- bis C₄- Polyhydroxyalkylgruppe oder ein Halogenatom.

Unter Keratinfasern sind dabei erfindungsgemäß Pelze, Wolle, Federn und insbesondere menschliche Haare zu verstehen. Obwohl die erfindungsgemäßen Oxidationsfärbemittel in erster Linie zum Färben von Keratinfasern geeignet sind, steht prinzipiell einer Verwendung auch auf anderen Gebieten, insbesondere in der Farbphotographie, nichts entgegen.

Da es sich bei den erfindungsgemäßen Farbstoffvorprodukten um Amino-Verbindungen handelt, lassen sich aus diesen in üblicher Weise die bekannten Säureadditionssalze herstellen. Alle Aussagen dieser Schrift und demgemäss der beanspruchte Schutzbereich beziehen sich daher sowohl auf die in freier Form vorliegenden Verbindungen als auch auf deren wasserlösliche, physiologisch verträgliche Salze. Beispiele für solche Salze sind die Hydrochloride, die Hydrobromide, die Sulfate, die Phosphate, die Acetate, die Propionate, die Citrate und die Lactate. Die Hydrochloride und die Sulfate sind dabei besonders bevorzugt.

Beispiele für die als Substituenten in den erfindungsgemäßen Verbindungen genannten C₁- bis C₄-AlkylGruppen sind die Gruppen Methyl, Ethyl, Propyl, Isopropyl und Butyl. Ethyl und Methyl sind bevorzugte Alkylreste. Bevorzugte C₁- bis C₄-Alkoxygruppen sind die Gruppen Methoxy und Ethoxy. Weiterhin können als bevorzugte Beispiele für eine C₁- bis C₄-Monohydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt.

Bevorzugt sind Verbindungen der Formel (I), bei denen R¹, R² und R³ für ein Wasserstoffatom stehen. Weiterhin sind die Verbindungen bevorzugt, bei denen R⁴ und R⁵ stehen unabhängig voneinander für ein Wasserstoffatom, eine Methylgruppe, ein Chlor- oder ein Fluoratom. Verbindungen der Formel (I), bei denen R⁴ und R⁵ für ein Wasserstoffatom stehen, sind besonders bevorzugt.

Ein wesentliches Merkmal der erfindungsgemäßen Verbindungen der Formel (I) ist der gegebenenfalls substituierte Imidazolylrest. In einer ersten bevorzugten Ausführungsform der vorliegenden Erfindung, steht die Gruppe X für einen gegebenenfalls substituierten Imidazolylrest der Formel (II) 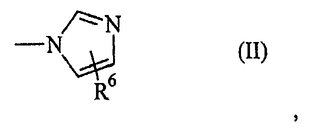 wobei R⁶ steht für ein Wasserstoffatom, eine C₁- bis C₄-Alkylgruppe, eine C₁- bis C₄-Monohydroxyalkylgruppe, eine C₂- bis C₄- Polyhydroxyalkylgruppe oder ein Halogenatom.

Im Rahmen dieser Ausführungsform sind Verbindungen der Formel (I) besonders bevorzugt, bei denen A steht für eine unverzweigte Alkylengruppe mit 2 bis 6 Kohlenstoffatomen. Eine besonders bevorzugte Alkylengruppe ist im Rahmen dieser Ausführungsform die Trimethylengruppe.

Ganz besonders bevorzugte Verbindung der Formel (I) mit einem Imidazolylrest der Formel (II) ist (4-Aminophenyl)(3-(imidazol-1-yl)propyl)amin (E2) der Formel (IIa);

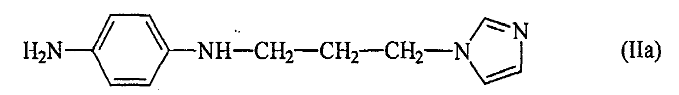

Ebenso bevorzugte Verbindung des Formel (I) ist (4-Amino-3-methylphenyl)(3-(imidazol-1-yl)propyl)amin (E3) der Formel (IIb).

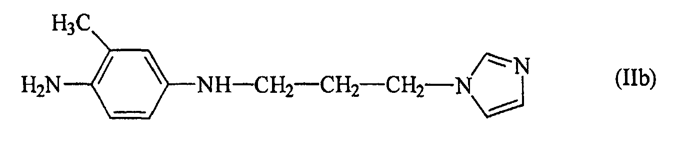

Im Rahmen einer zweiten bevorzugten Ausführungsform der vorliegenden Erfindung handelt es sich bei dem gegebenenfalls substituierten Imidazolylrest X und eine Gruppe der Formel (III) 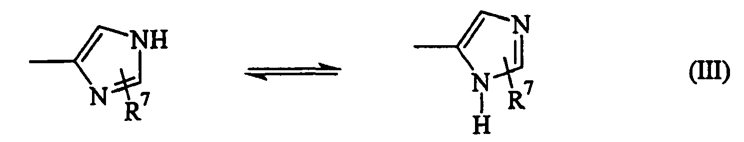 wobei R⁷ steht für ein Wasserstoffatom, eine C₁- bis C₄-Alkylgruppe, eine C₁- bis C₄-Monohydroxyalkylgruppe, eine C₂- bis C₄- Polyhydroxyalkylgruppe oder ein Halogenatom. Da die Verbindungen dieser Ausführungsform der vorliegenden Erfindung in Form eines tautomeren Gleichgewichtes vorliegen, beziehen sich die Aussagen dieser Schrift auf beide im Gleichgewicht vorliegenden Tautomere.

Im Rahmen dieser Ausführungsform der vorliegenden Erfindung steht die Gruppe A bevorzugt für eine unverzweigte Alkylengruppe mit 1 bis 6 Kohlenstoffatomen. Besonders bevorzugter Weise ist die Gruppe A eine Ethylengruppe.

Eine ganz besonders bevorzugte Verbindung der Formel (I) mit einem Imidazolylrest der Formel (III) ist (4-Aminophenyl)(2-(imidazol-5-yl)ethyl)amin (E1) der Formel (IIIa)

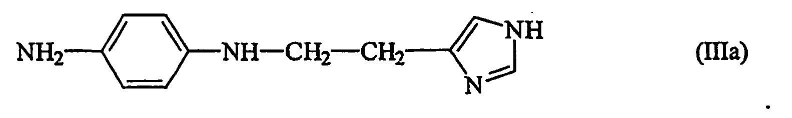

Neben den erfindungsgemäßen Verbindungen der Formel (I), können die Färbemittel ein oder mehrere weitere Farbstoffvorprodukte enthalten.

Hinsichtlich der in den erfindungsgemäßen Färbemitteln einsetzbaren weiteren Farbstoffvorprodukte unterliegt die vorliegende Erfindung keinerlei Einschränkungen. Die erfindungsgemäßen Färbemittel können als weitere Farbstoffvorprodukte
- Oxidationsfarbstoffvorprodukte vom Entwickler- und/oder Kuppler-Typ, und
- Vorstufen naturanaloger Farbstoffe, wie Indol- und Indolin-Derivate,
sowie Mischungen von Vertretern dieser Gruppen enthalten.

In einer ersten bevorzugten Ausführungsform enthält das Färbemittel mindestens eine weitere Entwicklerkomponente. Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolderivate sowie 2,4,5,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1) 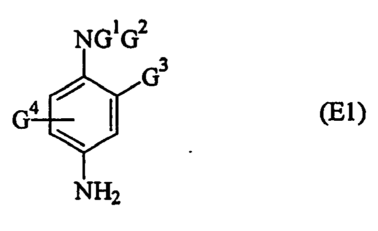 wobei
- G¹ steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen 4'-Aminophenylrest oder einen C₁- bis C₄-Alkylrest, der mit einer stickstoffhaltigen Gruppe, einem Phenyl- oder einem 4'-Aminophenylrest substituiert ist;
- G² steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest oder einen C₁- bis C₄-Alkylrest, der mit einer stickstoffhaltigen Gruppe substituiert ist;
- G³ steht für ein Wasserstoffatom, ein Halogenatom, wie ein Chlor-, Brom-, Iod- oder Fluoratom, einen C₁- bis C₄-Alkylrest, einen C₁- bis C₄-Monohydroxyalkylrest, einen C₂- bis C₄-Polyhydroxyalkylrest, einen C₁- bis C₄-Hydroxyalkoxyrest, einen C₁- bis C₄-Acetylaminoalkoxyrest, einen C₁- bis C₄- Mesylaminoalkoxyrest oder einen C₁- bis C₄-Carbamoylaminoalkoxyrest;
- G⁴ steht für ein Wasserstoffatom, ein Halogenatom oder einen C₁- bis C₄-Alkylrest oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden.

Beispiele für die als Substituenten in den erfindungsgemäßen Verbindungen genannten C₁- bis C₄-Alkylreste sind die Gruppen Methyl, Ethyl, Propyl, Isopropyl und Butyl. Ethyl und Methyl sind bevorzugte Alkylreste. Erfindungsgemäß bevorzugte C₁- bis C₄-Alkoxyreste sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (E1) sind insbesondere die Aminogruppen, C₁- bis C₄-Monoalkylaminogruppen, C₁- bis C₄-Dialkylaminogruppen, C₁- bis C₄-Trialkylammoniumgruppen, C₁- bis C₄-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze: 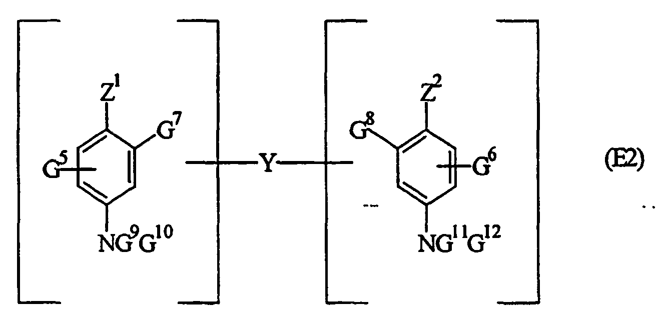 wobei:
- Z¹ und Z² stehen unabhängig voneinander für einen Hydroxyl- oder NH₂-Rest, der gegebenenfalls durch einen C₁- bis C₄-Alkylrest, durch einen C₁- bis C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁- bis C₈-Alkoxyreste substituiert sein kann, oder eine direkte Bindung,
- G⁵ und G⁶ stehen unabhängig voneinander für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen C₁- bis C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁- bis C₄-Alkylrest,
mit den Maßgaben, dass
- die Verbindungen der Formel (E2) nur eine Verbrückung Y pro Molekül enthalten und
- die Verbindungen der Formel (E2) mindestens eine Aminogruppe enthalten, die mindestens ein Wasserstoffatom trägt.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (E2) sind N,N'-Bis-(β-hydroxyethyl)-N,N'-bis-(4'-aminophenyl)-1,3-diamino-propan-2-ol, Bis-(2-hydroxy-5-aminophenyl)-methan, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 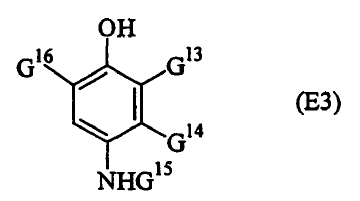 wobei:
- G¹³ steht für ein Wasserstoffatom, ein Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Akoxy-(C₁- bis C₄)-alkylrest, einen C₁- bis C₄-Aminoalkylrest, einen Hydroxy-(C₁- bis C₄)-alkylaminorest, einen C₁- bis C₄-Hydroxyalkoxyrest, einen C₁- bis C₄-Hydroxyalkyl-(C₁-bis C₄)-aminoalkykest oder einen (Di-C₁- bis C₄-Alkylamino)-(C₁- bis C₄)-alkylrest, und
- G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest oder einen C₁- bis C₄-Cyanoalkylrest,
- G¹⁵ steht für Wasserstoff, einen C₁- bis C₄-Alkylrest, einen C₁- bis C₄-Monohydroxyalkylrest, einen C₂- bis C₄-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4'-Methoxyphenyl)-amino-3-amino-pyridin, 2,3-Diamino-6-methoxy-pyridin, 2-(β-Methoxyethyl)-amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740 931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amimo-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)-armno-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)-amino-l-methylpyrazol.

Bevorzugte Pyrazol-Pyrimidin-Derivate sind insbesondere die Derivate des Pyrazol-[1,5-a]-pyrimidin der folgenden Formel (E4) und dessen tautomeren Formen, sofern ein tautomeres Gleichgewicht besteht: 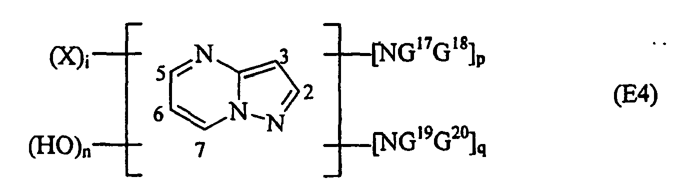 wobei:
- G¹⁷, G¹⁸, G¹⁹ und G²⁰ unabhängig voneinander stehen für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest einen (C₁- bis C₄)-Alkoxy-(C₁- bis C₄)-alkylrest, einen C₁- bis C₄-Aminoalkylrest, der gegebenenfalls durch ein Acetyl-Ureid- oder einen SulfonylRest geschützt sein kann, einen (C₁- bis C₄)-Alkylamino-(C₁- bis C₄)-alkylrest, einen Di-[(C₁- bis C₄)-alkyl]-(C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄)-[Hydroxyalkyl]-(C₁- bis C₄)-aminoalkylrest,
- die X-Reste stehen unabhängig voneinander für ein Wasserstoffatom, einen C₁- bis C₄-Alkylrest, einen Aryl-Rest, einen C₁- bis C₄-Hydroxyalkykest, einen C₂- bis C₄-Polyhydroxyalkylrest, einen C₁- bis C₄-Aminoalkylrest, einen (C₁- bis C₄)-Alkylamino-(C₁- bis C₄)-alkylrest, einen Di-[(C₁- bis C₄)alkyl]- (C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄-hydroxyalkyl)-aminoalkylrest, einen Aminorest, einen C₁- bis C₄-Alkyl- oder Di-(C₁- bis C₄-hydroxyalkyl)-aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
mit der Maßgabe, dass
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (E4) verwendeten Substituenten sind erfindungsgemäß analog zu den obigen Ausführungen definiert.

Wenn das Pyrazol-[1,5-a]-pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird:

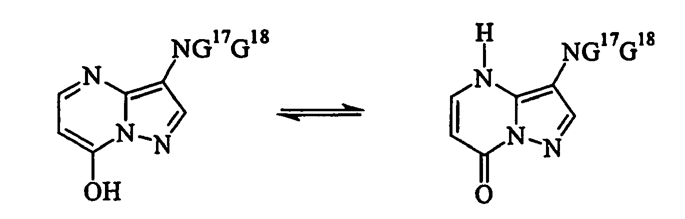

Unter den Pyrazol-[1,5-a]-pyrimidinen der obenstehenden Formel (E4) kann man insbesondere nennen:
- Pyrazol-[1,5-a]-pyrimidin-3,7-diamin;
- 2,5-Dimethyl-pyrazol-[1,5-a]-pyrimidin-3,7-diamin;
- Pyrazol-[1,5-a]-pyrimidin-3,5-diamin;
- 2,7-Dimethyl-pyrazol-[1,5-a]-pyrimidin-3,5-diamin;
- 3-Aminopyrazol-[1,5-a]-pyrimidin-7-ol;
- 3-Aminopyrazol-[1,5-a]-pyrimidin-5-ol;
- 2-(3-Aminopyrazol-[1,5-a]-pyrimidin-7-ylamino)-ethanol;
- 2-(7-Aminopyrazol-[1,5-a]-pyrimidin-3-ylamino)-ethanol;
- 2-[(3-Aminopyrazol-[1,5-a]-pyrimidin-7-yl)-(2-hydroxy-ethyl)-amino]-ethanol;
- 2-[(7-Aminopyrazol-[1,5-a]-pyrimidin-3-yl)-(2-hydroxy-ethyl)-amino]-ethanol;
- 5,6-Dimethylpyrazol-[1,5-a]-pyrimidin-3,7-diamin;
- 2,6-Dimetbylpyrazol-[1,5-a]-pyrlmidin-3,7-diamin;
- 3-Amino-7-dimethylamino-2,5-dimethylpyrazol-[1,5-a]-pyrimidin;
sowie ihre physiologisch verträglichen Salze und ihre tautomeren Formen, wenn ein tautomers Gleichgewicht vorhanden ist.

Die Pyrazol-[1,5-a]-pyrimidine der obenstehenden Formel (E4) können wie in der Literatur beschrieben durch Zyklisierung ausgehend von einem Aminopyrazol oder von Hydrazin hergestellt werden.

In einer zweiten bevorzugten Ausführungsform enthalten die erfindungsgemäßen Färbemittel mindestens eine Kupplerkomponente.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Erfindungsgemäß bevorzugte Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer zweiten bevorzugten Ausführungsform enthalten die Färbemittel mindestens ein Indol- und/oder Indolinderivat.

Besonders gut als Vorstufen naturanaloger Haarfarbstoffe geeignet sind Derivate des 5,6-Dihydroxyindolins der Formel (IVa), 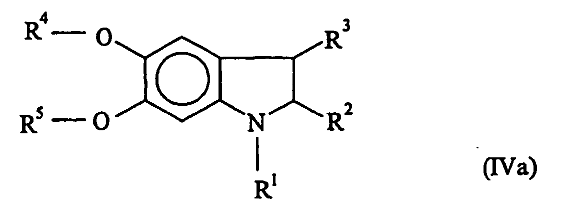 in der unabhängig voneinander
- R¹ steht für Wasserstoff, eine C₁-C₄-Alkylgruppe oder eine C₁-C₄-Hydroxy-alkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoiadolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (IVb), 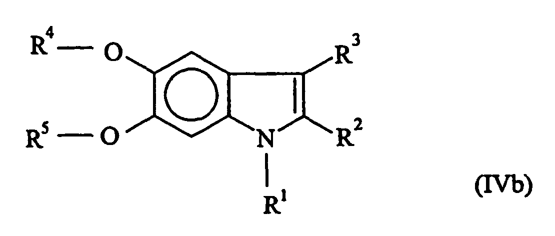 in der unabhängig voneinander
- R¹ steht für Wasserstoff, eine C₁-C₄-Alkylgruppe oder eine C₁-C₄-Hydroxyalkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroayindol sowie insbesondere das 5,6-Dihydroxyindol.

Die Indolin- und Indol-Derivate können in den im Rahmen des erfindungsgemäßen Verfahrens eingesetzten Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.%, vorzugsweise 0,2-5 Gew.-% enthalten.

In einer weiteren Ausführungsform kann es erfindungsgemäß bevorzugt sein, das Indolin- oder Indolderivat in Haarfärbemitteln in Kombination mit mindestens einer Aminosäure oder einem Oligopeptid einzusetzen. Die Aminosäure ist vorteilhafterweise eine α-Aminosäure; ganz besonders bevorzugte α-Aminosäuren sind Arginin, Ornithin, Lysin, Serin und Histidin, insbesondere Arginin.

Neben den weiteren Farbstoffvorprodukten können die erfindungsgemäßen Färbemittel zur Nuancierung einen oder mehrere direktziehende Farbstoffe enthalten. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, HC Orange 1, Disperse Orange 3, HC Red 1, HC Red 3, HC Red 13, HC Red BN, Basic Red 76, HC Blue 2, HC Blue 12, Disperse Blue 3, Basic Blue 7, Basic Blue 26, Basic Blue 99, HC Violet 1, Disperse Violet 1, Disperse Violet 4, Basic Violet 2, Basic Violet 14, Acid Violet 43, Disperse Black 9, Acid Black 52, Basic Brown 16 und Basic Brown 17 bekannten Verbindungen sowie 1,4-Bis-(β-hydroxyethyl)-amino-2-nitrobenzol, 3-Nitro-4-(β-hydroxyethyl)-aminophenol, 4-Amino-2-nitrodiphenylamin-2'-carbonsäure, 6-Nitro-1,2,3,4-tetrahydrochinoxalin, 2-Hydroxy-1,4-naphthochinon, Hydroxyethyl-2-nitro-toluidin, Pikraminsäure, 2-Amino-6-chloro-4-nitrophenol, 4-Ethylamino-3-nitrobenzoesäure und 2-Chloro-6-ethylamino-1-hydroxy-4-nitrobenzol.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Die erfindungsgemäßen Färbemittel können weiterhin alle für solche Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkauolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH_{2O})ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R¹O-(Z)ₓ. Diese Verbindungen sind durch die folgenden Parameter gekennzeichnet.

Der Alkylrest R¹ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R¹
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, dass eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten..

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten.

Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethylammoniumchlorid, sowie Dehyquart® F-75 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyl-dimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Ein Beispiel für ein als kationisches Tensid einsetzbares quaternäres Zuckerderivat stellt das Handelsprodukt Glucquat^{®} 100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Ferner können die erfindungsgemäßen Färbemittel weitere Wirk-, Hilfs- und Zusatzstoffe, wie beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobomylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöl, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrulcturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O, Dimethylether, CO₂ und Luft,
- Antioxidantien,
enthalten

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die erfindungsgemäßen Mittel enthalten die Farbstoffvorprodukte bevorzugt in einem geeigneten wäßrigen, alkoholischen oder wäßrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch denkbar, die Farbstoffvorprodukte in eine pulverförmige oder auch Tablettenförmige Formulierung zu integrieren.

Unter wäßrig-alkoholischen Lösungen sind im Sinne der vorliegenden Erfindung wäßrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Die eigentliche oxidative Färbung der Fasern kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z.B. Metallionen, Iodide, Chinone oder bestimmte Enzyme.

Geeignete Metallionen sind beispielsweise Zₙ²⁺, Cᵤ²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Das eigentliche Haarfärbemittel wird zweckmäßigerweise unmittelbar vor der Anwendung durch Mischung der Zubereitung des Oxidationsmittels mit der Zubereitung, enthaltend die Farbstoffvorprodukte, hergestellt. Das dabei entstehende gebrauchsfertige Haarfärbepräparat sollte bevorzugt einen pH-Wert im Bereich von 6 bis 12 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Insbesondere bei schwer färbbarem Haar kann die Zubereitung mit den Farbstoffvorprodukten aber auch ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Ein zweiter Gegenstand der vorliegenden Anmeldung ist die Verwendung der erfindungsgemäßen Mittel zum Färben keratinischer Fasern.

Ein dritter Gegenstand der vorliegenden Anmeldung ist ein Verfahren zur Färbung keratinischer Fasern, bei dem ein erfindungsgemäßes Mittel auf die Fasern aufgetragen wird und nach einer Einwirkzeit wieder abgespült wird.

Ein vierter Gegenstand der vorliegenden Erfindung sind p-Phenylendiatninderivate der Formel (I) 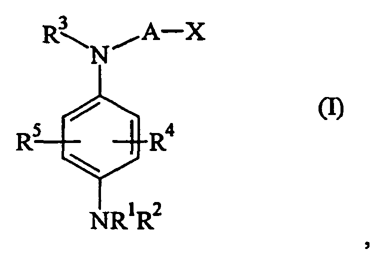 bei denen
A steht für eine verzweigte oder unverzweigte Alkylengruppe mit 1 bis 6 Kohlenstoffatomen, die einen oder mehrere Substituenten, ausgewählt aus Hydroxygruppe(n) und Halogenatom(en), tragen kann,
X steht für einen gegebenenfalls substituierten Imidazolylrest der Formel (III), 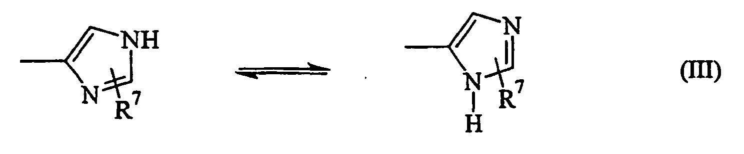
R¹, R² und R³ stehen unabhängig voneinander für ein Wasserstoffatom, eine C₁- bis C₄-Alkylgruppe, eine C₁- bis C₄-Monohydroxyalkylgruppe oder eine C₂- bis C₆-Polyhydroxyalkylgruppe,
R⁴ und R⁵ stehen unabhängig voneinander für ein Wasserstoffatom, eine C₁- bis C₄-Alkylgruppe, eine C₁- bis C₄-Alkoxygruppe,eine C₁- bis C₄-Monohydroxyalkylgruppe, eine C₂- bis C₄- Polyhydroxyalkylgruppe oder ein Halogenatom und
R⁷ steht für ein Wasserstoffatom, eine C₁- bis C₄-Alkylgruppe, eine C₁- bis C₄-Monohydroxyalkylgruppe, eine C₂- bis C₄- Polyhydroxyalkylgruppe oder ein Halogenatom.

Ein besonders bevorzugtes p-Phenylendiaminderivat der Formel (I) ist (4-Aminophenyl)(2-(imidazol-5-yl)ethyl)amin der Formel (IIIa).

### Ausführungsbeispiele

### A Herstellung der Verbindungen

### 1. Herstellung von (4-Aminophenyl)(2-(imidazol-5-yl)ethyl)amin

### 1.1 Herstellung von (4-Nitrophenyl)(2-(imidazol-5-yl)ethyl)amin

In einem 500 ml Dreihalskolben wurden 100 ml DMSO vorgelegt und mit 30,9g Triethylamin, 18,4g Histamin-dihydrochlorid und 14,4g 1-Fluor-4-nitrobenzol versetzt. Die Mischung wurde 10h bei 80°C gerührt, mit 20 ml Wasser versetzt und weitere 3h bei 80°C gerührt. Im Anschluss ließ man auf Raumtemperatur abkühlen und goß auf IL Eis. Der entstandene Niederschlag wurde abgesaugt und im Vakuum bei 50°C getrocknet, wobei 20,8g Rohprodukt erhalten wurden.

Zwecks Aufreinigung wurden 20,1g Rohprodukt dreimal mit je 400ml Aceton ausgekocht und filtriert, wobei der unlöslich Rückstand verworfen wurde. Die vereinigten Aceton-Lösungen wurden auf insgesamt 400ml eingeengt, und über Nacht bei 5°C auslaistallisiert. Die erhaltenen Kristalle wurden im Vakuum bei 50°C getrocknet.

| | |
|---|---|
| Ausbeute: | 15,2 g (65 °C) |
| Schmelzpunkt: | 177-223 °C |

### 1.2 Herstellung von (4-Aminophenyl)(2-(imidazol-5-yl)ethyl)amin-trihydrochlorid (E1)

14,4g (4-Nitrophenyl)(2-(imidazol-5-yl)ethyl)amin wurden in 400ml Ethanol gelöst, mit 1,0g Pd/C (5%ig) versetzt und in einer Wasserstoff-Schüttelapparatur bei Raumtemperatur und Normaldruck katalytisch reduziert. Nach beendeter Wasserstoff-Aufnahme wurde mit 100ml 2n Salzsäure versetzt, filtriert und im Vakuum bis zur Trockne eingeengt. Im Anschluss wurde im Vakuum bei 50 °C getrocknet.

| | |
|---|---|
| Ausbeute: | 18,6 g (96%) |
| Schmelzpunkt: | > 179 °C (Zersetzung) |

### 2. Herstellung von (4-Aminophenyl)(3-(imidazol-1-yl)propyl)amin

### 2.1 Herstellung von (4-Nitrophenyl)(3-(imidazol-1-yl)propyl)amin

In einem 500 ml Dreihalskolben wurden 100ml DMSO vorgelegt und mit 10,3g Triethylamin, 12,8g 1-(3-Aminopropyl)imidazol und 14,4g 1-Fluor-4-nitrobenzol versetzt. Die Mischung wurde 12h bei 80°C gerührt, auf Raumtemperatur abgekühlt und anschließend auf 1L Eis gegossen. Vom entstandenen öligen Niederschlag wurde eine Probe mit H₂O verrieben bis Kristallisation eintritt. Mit den so gewonnenen Kristallen wurde angeimpft und 1h bei Raumtemperatur kristallisiert. Der entstandene Niederschlag wurde abgesaugt, mit H₂O verrieben, erneut abgesaugt und mit H₂O nachgewaschen. Zwecks Aufreinigung worden die so erhaltenen 28g feuchtes Rohprodukt aus 250ml H₂O und 114ml Ethanol unkristallisiert. Das Produkt wurde im Vakuum bei 50 °C getrocknet.

| | |
|---|---|
| Ausbeute: | 20,1 g (81%) |
| Schmelzpunkt: | 130-132 °C |

### 2.2 Herstellung von (4-Aminophenyl)(3-(imidazol-1-yl)propyl)amin-trihydrochlorid (E2)

14.4g (4-Nitrophenyl)(3-imidazolylpropyl)amin wurden in 400ml Ethanol gelöst, mit 1,5g Pd/C (5%ig) versetzt und in einer Wassemoff-Schüttelapparatur bei Raumtemperatur und Normaldruck katalytisch induziert. Nach beendeter Wasserstoff-Aufnahme wurde mit 130ml 2n Salzsäure versetzt, filtriert und im Vakuum bis zur Trockne eingeengt, wobei mehrfach wieder mit Ethanol gelöst wurde. Das so erhaltene Öl wurde nach 4 Tagen bei 5 °C teilkristallin. Nach einer Behandlung mit Methyl-tert.-butylether wurde dekantiert. Im Auschluss wurde im Vakuum bei 50 °C getrocknet.

| | |
|---|---|
| Ausbeute: | 22,1 g (90%) |
| Schmelzpunkt: | > 231 °C (Zersetzung) |

### 3. Herstellung von (4-Amino-3-methylphenyl)(3-imidazol-1-ylpropyl)amintrihydrochlorid (E3)

### 3.1 Herstellung von (4-Nitro-3-methylphenyl)(3-(imidazol-1-yl)propyl)amin

In einem 500 ml Dreihalskolben wurden 150 ml Dimethylsulfoxid vorgelegt und mit 22,6 g Triethanolamin (99 %ig), 28,7 g 1-(3-Aminopropyl)imidazol (98 %ig) und 24,2 g 5-Fluor-2-nitrotoluol (96 %ig) versetzt. Die Mischung wurde 17 h bei 80 °C gerührt. Im Anschluss wurde der Ansatz auf Raumtemperatur abgekühlt und auf 1 1 Eis gegossen, wobei ein gelbes Öl ausfiel, welches nach ca. 1 h durchkristallisierte. Der so entstandene Niederschlag wurde abgesaugt, mit Wasser gewaschen und im Vakuum bei 40 °C getrocknet.

| | |
|---|---|
| Ausbeute: | 38 g (98 %) |
| Schmelzpunkt: | 135 - 138 °C |

### 3.2 Herstellung von (4-Amino-3-methylphenyl)(3-imidazol-1-ylpropyl)amintrihydrochlorid (E3)

36,4 g N-(3-Imidazol-1-yl-propyl)-4-nitro-anilin wurden in 400 ml Ethanol suspendiert, mit 2,5 g Pd/C (5 %ig) versetzt und in einer Wasserstoff-Schüttelapparatur 3 h katalytisch reduziert. Nach beendeter Wasserstoff-Aufnahme wurde mit 200 ml verdünnter HCl versetzt, der Rückstand abfiltriert und das Filtrat bis zur Trockne eingeengt.

| | |
|---|---|
| Ausbeute: | 42 g (88 %) |
| Schmelzpunkt: | 155 °C (Zersetzung) |

### B Ausfärbungen

Für die nachfolgend beschriebenen Ausfärbungen wurden die vorhergehend beschriebenen erfindungsgemäßen Entwicklerkomponenten E1, E2 und E3 verwendet.

### 1. Ausfärbungen aus einer Standardfärbecreme

Für die Herstellung der Färbecreme wurden 50g einer Cremebasis in einem 250ml Becherglas eingewogen und bei 80°C geschmolzen. Die verwendete Cremebasis hatte die folgende Zusammensetzung:

| | |
|---|---|
| Hydrenol® D¹ | 17,0 Gew.-% |
| Lorol® tech.² | 4,0 Gew.-% |
| Texapon^{®} NSO³ | 40,0 Gew.-% |
| Dehyton^{®} K⁴ | 25,0 Gew.-% |
| Eumulgin® B2⁵ | 1,5 Gew.-% |
| Wasser | 12,5 Gew.-% |

| | |
|---|---|
| ¹ C₁₆₋₁₈-Fettakohol (INCI-Bezeichnung: Cetearyl alcohol) (Cognis) ² C₁₂₋₁₈-Fettalkohol (INCI-Bezeichnung: Coconut alcohol) (Cognis) ³ Laurylethersulfat, Natriumsalz (ca. 27,5% Aktivsubstanz; INCI-Bezeichnung: Sodium Laureth Sulfate) (Cognis) ⁴ N,N-Dimetliyl-N-(C₈₋₁₈-kokosamidopropyl)ammoniumacetobetain (ca. 30% Aktivsubstanz; INCI-Bezeichnung: Aqua (Water), Cocamidopropyl Betaine) (Cognis) ⁵ Cetylstearylalkohol mit ca. 20 EO-Einheiten (INCI-Bezeichnung: Ceteareth-20) (Cognis) | |

Die Farbstoffvorprodukte wurden jeweils getrennt in destilliertem Wasser suspendiert bzw. unter Erwärmen gelöst. Anschließend wurde Ammoniak (<1 ml; 25%ige Ammoniaklösung) zugegeben bis der pH-Wert zwischen 9 und 10 lag. Durch die Zugabe von Ammoniak entstand eine Lösung.

Die gelösten Farbstoffvorprodukte wurden nacheinander in die heiße Creme eingearbeitet. Anschließend wurde mit destilliertem Wasser auf 97g aufgefüllt und mit Ammoniak ein pH-Wert von 9,5 eingestellt. Nach Auffüllen mit destilliertem Wasser auf 100g wurde der Ansatz kaltgerührt (< 30°C), wobei eine homogene Creme entstand.

Die Creme wurde für die unterschiedlichen Ausfärbungen wie folgt verdünnt:

| | |
|---|---|
| Luftoxidation: | 25g Creme + 25g destilliertes Wasser |
| Oxidation mit 1 Gew.-% H₂O₂ | 25g Creme + 25g wässrige 1Gew.-%ige H₂O₂-Lösung |
| Oxidation mit 3 Gew.-% H₂O₂ | 25g Creme + 25g wässrige 3Gew.-%ige H₂O₂-Lösung |
| Oxidation mit 9 Gew.-% H₂O₂ | 25g Creme + 25g wässrige 9Gew.-%ige H₂O₂-Lösung |

In jede der so erhaltenen Mischungen wurde eine Haarsträhnen (80% ergraut; 330mg bis 370mg schwer) gegeben. Anschließend wurden die Mischungen und die Haarsträhnen auf jeweils ein Uhrglas gegeben und die Haarsträhnen in die Färbecremes gut eingebettet. Nach 30 Minuten (±1 Minute) Einwirkzeit bei Raumtemperatur wurden die Haarsträhnen entnommen und mit einer wässrigen Texapon® EVR-Lösung⁶ so oft gewaschen, bis der Farbüberschuß entfernt war. Die Haarsträhnen wurden an der Luft getrocknet und ihr Farbton unter der Tageslichtlampe (Farbprüfgerät HE240A) bestimmt und notiert (Taschenlexikon der Farben, A. Kornerup u. J.H. Wanscher, 3. unveränderte Auflage 1981, MUSTER-SCHMIDT Verlag; Zürich, Göttingen).
⁶ Laurylethersulfat-Natrium-Salz mit speziellen Zusätzen (ca. 34 bis 37% Aktivsubstanzgehalt; INCI-Bezeichnung: Sodium Lauryl Sulfate, Sodium Laureth Sulfate, Lauramide MIPA, Cocamide MEA, Glycol Stearate, Laureth-10) (Cognis)

Die bei den Ausfärbungs-Untersuchungen erhaltenen Ergebnisse sind in den nachstehenden Tabellen aufgeführt.

### 1.1 Ausfärbungen mit Kupplern

Bei den folgenden Ausfärbungen wurden die erfindungsgemäßen Entwicklerkomponenten in einem molaren Verhältnis von 1:1 zu den Kupplerkomponenten eingesetzt. Es wurden jeweils 1/400 Mol der Entwickler- bzw. Kupplerkomponente eingesetzt.

Es wurden die folgenden Kupplerkomponenten eingesetzt:
- K1:: Resorcin
- K2:: 3-Amino-6-methoxy-2-(methylamino)pyridin x 2 HCl
- K3:: 5-Amino-2-methylphenol
- K4:: 2-Amino-3-hydroxypyridin
- K5:: 1,3-Bis(2,4-diaminophenoxy)propan x 4 HCl
- K6:: 2,7-Dihydroxynaphthalin
- K7:: 1-Hydroxynaphthalin

### 1.1.1 Ausfärbungen mit Luftoxidation

| Kuppler | Entwickler | |
|---|---|---|
| | E1 | E2 |
| K1 | olivbraun | nougatfarbig |
| K2 | schmutziggrau | olivbraun |
| K3 | purpurgrau | graubraun |
| K4 | violettgrau | violettgrau |
| K5 | graublau | mattblau |
| K6 | flachsgelb | braunorange |

### 1.1.2 Ausfärbungen mit 1%iger wässriger H₂O₂-Lösung

| Kuppler | Entwickler | |
|---|---|---|
| | E1 | E2 |
| K1 | graubraun | negerbraun |
| K2 | schwarzblau | schwarzblau |
| K3 | dunkelviolett | dunkelviolett |
| K4 | dunkelpurpur | dunkelpurpur |
| K5 | schwarzblau | schwarzblau |
| K6 | erdbraun | nutria |
| K7 | nicht bestimmt | marineblau |

### 1.1.3 Ausfärbungen mit 3%iger wässriger H₂O₂-Lösung

| Kuppler | Entwickler E3 |
|---|---|
| K1 | olivbraun |
| K2 | dunkelgrün |
| K3 | dunkelviolett |
| K4 | teakholzfarbig |
| K5 | dunkelblau |
| K6 | olivbraun |

### 1.1.4 Ausfärbungen mit 9%iger wässriger H₂O₂-Lösung

| Kuppler | Entwickler | |
|---|---|---|
| | E1 | E2 |
| K1 | braungrau | rußbraun |
| K2 | tintenblau | blaugrau |
| K3 | dunkelviolett | dunkelviolett |
| K4 | rotbraun | dunkelbraun |
| K5 | schwarzblau | schwarzblau |
| K6 | mausgrau | oliv |

### 1.2 Ausfärbungen des erfindungsgemäßen Entwicklers E2 mit einem weiteren Entwickler sowie einer Kupplerkomponente

Bei den folgenden Ausfärbungen wurden die Mengenverhältnisse der verschiedenen Komponenten derart gewählt, dass das Verhältnis der beiden Entwicklerkomponenten 1:1 und das Verhältnis der Summe der Entwicklerkomponenten zur Kupplerkomponente ebenfalls 1:1 betrug. Es wurden insgesamt jeweils 1/400 Mol der Entwicklerkomponenten und 1/400 Mol der Kupplerkomponente eingesetzt. Die Kupplerkomponenten sind wie oben angegeben definiert.

Es wurden die folgenden weiteren Entwicklerkomponenten eingesetzt:
E4: 1-(β-Hydroxyethyl)-2,5-diaminobenzol · H₂SO₄
E5: 3-Methyl-4-aminophenol
E6: 2,4,5,6-Tetraaminopyrimidin · H₂SO₄
E7: 1-(β-Hydroxyethyl)-4,5-diaminopyrazol · H₂SO₄

### 1.2.1 Ausfärbungen mit Luftoxidation

| Kuppler | Weitere Entwicklerkomponente | | | |
|---|---|---|---|---|
| | E4 | E5 | E6 | E7 |
| K1 | braunorange | nougatfarbig | hellbraun | mattrot |
| K2 | mausgrau | olivbraun | dunkelgrün | braungrau |
| K3 | braungrau | graubraun | mattviolett | rotbraun |
| K4 | braungrau | mausgrau | braungrau | braungrau |
| K5 | blaugrau | blaugrau | tiefblau | dunkelblau |
| K6 | nougatfarbig | flachsgelb | lehmfarbig | nougatfarbig |

### 1.2.2 Ausfärbungen mit 1%iger wässriger H₂O₂-Lösung

| Kuppler | Weitere Entwicklerkomponente | | | |
|---|---|---|---|---|
| | E4 | E5 | E6 | E7 |
| K1 | negerbraun | haarbraun | dunkelbraun | violettbraun |
| K2 | schwarzblau | dunkelgrün | dschungelgrün | dunkelviolett |
| K3 | dunkelpurpur | graurubin | dunkelviolett | violettbraun |
| K4 | rotbraun | rotbraun | graubraun | portweinrot |
| K5 | schwarzblau | schwarzblau | schwarzblau | dunkelviolett |
| K6 | braungrau | fahl | bronzino | haarbraun |

### 1.23 Ausfärbungen mit 9%iger wässriger H₂O₂-Lösung

| Kuppler | Weitere Entwicklerkomponenten | | | |
|---|---|---|---|---|
| | E4 | E5 | E6 | E7 |
| K1 | schokoladebraun | olivbraun | dunkelbraun | leberbraun |
| K2 | blaugrau | nickelgrün | dunkelgrün | dunkelviolett |
| K3 | dunkelpurpur | graurubin | dunkelviolett | violettbraun |
| K4 | somali | rehbraun | braun | dunkelbraun |
| K5 | schwarzblau | schwarzblau | dunkelblau | dunkelviolett |
| K6 | nutria | fahl | olivbraun | olivbraun |

### 1.3 Ausfärbungen des erfindungsgemäßen Entwicklers E1 mit einem weiteren Entwickler sowie Standardkupplern

Bei den folgenden Ausfärbungen wurden die Mengenverhältnisse der verschiedenen Komponenten derart gewählt, dass das Verhältnis der beiden Entwicklerkomponenten 1:1 und das Verhältnis der Summe der Entwicklerkomponenten zur Kupplerkomponente ebenfalls 1:1 betrug. Es wurden insgesamt jeweils 1/400 Mol der Entwicklerkomponenten und 1/400 Mol der Kupplerkomponente eingesetzt.

Die Entwicklerkomponenten und Kupplerkomponenten sind wie oben angegeben definiert.

### 1.3.1 Ausfärbungen mit 6%iger wässriger H₂O₂-Lösung

| Kuppler | Weitere Entwicklerkomponente | | | |
|---|---|---|---|---|
| | E4 | E5 | E6 | E7 |
| K1 | braunbeige | mausgrau | dunkelbraun | rotbraun |
| K2 | tintenblau | dunkelgrün | oliv | schwarzblau |
| K3 | dunkelpurpur | violettbraun | burgunderrot | violettbraun |
| K4 | somali | rehbraun | somali | rotbraun |
| K5 | schwarzblau | schwarzblau | dunkelgrün | dunkelviolett |
| K6 | olivbraun | oliv | oliv | olivbraun |

### 2. Ausfärbung aus marktrelevanten Färbecremes

Die folgenden Rezepturen wurden vor der Anwendung im Gewichtsverhältnis 1:1 mit einer Oxidationsmittelzubereitung (Rezeptur 2.14) vermischt. Die resultierenden Anwendungszubereitungen wurden jeweils auf Strähnen (Kerling Naturweiß) aufgetragen, dort für 30 Minuten bei 32 °C belassen und anschließend gründlich ausgespült.

### Rezeptur 2.1

| Rohstoff | Menge in Gew.-% |
|---|---|
| Fettalkoholgemisch C₁₂-C₁₈ | 6,0 |
| Eumulgin® B2 | 0,5 |
| Texapon® NSO | 10,0 |
| Dehyton® K | 5,0 |
| Polymer JR® 400⁷ | 0,4 |
| Gafquat® 755 N⁸ | 0,2 |
| Celquat® L 200⁹ | 0,2 |
| Ascorbinsäure | 0,4 |
| Ammoniumsulfat | 0,5 |
| E2 (erfindungsgemäß) | 0,14 |
| Bis-(5-amino-2-hydroxyphenyl)methandihydrochlorid | 0,031 |
| 4-Amino-3-methylphenol | 0,011 |
| 2-Amino-3-hydroxypyridin | 0,006 |
| 3-Amino-2,4-dichlorphenol | 0,012 |
| 1,3-Bis-(2',4'-diaminophenoxy) propantetrahydrochlorid | 0,0004 |
| 1,3-Bis-(2',4'-diamiophenyl) propantetrahydrochlorid | 0,0004 |
| Resorcin | 0,015 |
| 4-Chlorresorcin | 0,015 |
| 3-Aminophenol | 0,003 |
| Ammoniak, 25 %ig | ad pH 9,8 |
| Wasser | ad 100 |
| Farbresultat | perlgrau |

### Rezeptur 2.2

| Rohstoff | Menge in Gew.-% |
|---|---|
| Fettalkoholgemisch C₁₂-C₁₈ | 6,0 |
| Eumulgin® B2 | 0,5 |
| Texapon® NSO | 10,0 |
| Dehyton® K | 5,0 |
| Polymer JR® 400 | 0,4 |
| Gafquat® 755 | 0,2 |
| Celquat® L 200 | 0,2 |
| Ascorbinsäure | 0,4 |
| Ammoniumsulfat | 0,5 |
| E3 (erfindungsgemäß) | 1,21 |
| Bis-(5-amino-2-hydroxyphenyl)methan-dihydrochlorid | 1,09 |
| 4-Amino-2-aminomethylphenol-dihydrochlorid | 0,05 |
| 4-Amino-2-((diethylamino)-methyl)-phenol-dihydrochlorid | 0,05 |
| 4-Amino-3-methylphenol | 0,30 |
| 2-Amino-4-methylphenol | 0,02 |
| 2-Amino-3-hydroxypyridin | 0,12 |
| 5-Amino-4-chlor-2-methylphenol | 0,12 |
| 2-Methylresorcin | 0,24 |
| 4-Chlorresorcin | 0,12 |
| 3-Aminophenol | 0,24 |
| 5,6-Dihydroxyindolin-hydrobromid | 0,06 |
| Ammoniak, 25 %ig | ad pH 9,8 |
| Wasser | ad 100 |
| Farbresultat | kakaobraun |

### Rezeptur 2.3

| Rohstoff | Menge in Gew.-% |
|---|---|
| Fettalkoholgemisch C₁₂-C₁₈ | 6,0 |
| Eumulgin® B2 | 0,5 |
| Texapon® NSO | 10,0 |
| Dehyton® K | 5,0 |
| Polymer JR® 400 | 0,4 |
| Gafquat® 755 | 0,2 |
| Celquat® L 200 | 0,2 |
| Ascorbinsäure | 0,4 |
| Ammoniumsulfat | 0,5 |
| E1 (erfindungsgemäß) | 0,03 |
| Bis-(5-amino-2-hydroxyphenyl)methan-dihydrochlorid | 1,36 |
| p-Toluylendiamin-sulfat | 0,03 |
| p-Phenylendiamin-dihydrochlorid | 0,03 |
| 2-(2'-Hydroxyethyl)-p-phenylendiamin-sulfat | 0,07 |
| N,N-Bis-(2`-Hydroxyethyl)-p-phenylendiamin-sulfat | 0,07 |
| 4-Amino-3-methylphenol | 0,55 |
| 2-Amino-5-methylphenol | 0,01 |
| 2-Amino-4-chlorphenol | 0,02 |
| 4-Amino-2-chlorphenol | 0,01 |
| 2-Amino-3-hydroxypyridin | 0,5 |
| 5-Anino-2-methylphenol | 0,03 |
| 5-(2`-Hydroxyethyl) amino-2-methylphenol | 0,01 |
| 3-Amino-2-chlor-6-methylphenol | 0,02 |
| 6-Hydroxyindol | 0,1 |
| 1,2,3,4-Tetrahydro-6-nitrochinoxalin | 0,05 |
| HC Yellow 5 | 0,03 |
| HC Red 1 | 0,01 |
| 4-Amino-2-nitro-diphenylamin-2'-carbonsäure | 0,01 |
| Ammoniak, 25 %ig | ad pH 9,8 |
| Wasser | ad 100 |
| Farbresultat | lederbraun |

### Rezeptur 2.4

| Rohstoff | Menge in Gew.-% |
|---|---|
| Fettalkoholgemisch C₁₂-C₁₈ | 10,0 |
| Texapon® K14 S70C¹⁰ | 2,5 |
| Plantaren® 1200 UP¹¹ | 2,0 |
| Akypo Soft® 45 NV¹² | 12,0 |
| Eutanol® G¹³ | 1,0 |
| Eumulgin® B1¹⁴ | 0,5 |
| Eumulgin® B2 | 0,5 |
| Polymer W 37194¹⁵ | 2,0 |
| Cosmedia Guar® C261¹⁶ | 0,2 |
| Mirapol® A15¹⁷ | 0,5 |
| Ascorbinsäure | 0,4 |
| Ammoniumsulfat | 0,5 |
| E1 (erfindungsgemäß) | 0,030 |
| Bis-(5-amino-2-hydroxyphenyl)methan-dihydrochlorid | 0,037 |
| 2-(2'-Hydroxyethyl)-p-phenylendiamin-sulfat | 0,022 |
| 1-Naphthol | 0,015 |
| 2-Methyl-1-naphthol | 0,022 |
| 1,5-Dihydroxynaphthalin | 0,005 |
| 3-Amino-2-methylamino-6-methoxypyridin | 0,003 |
| 2-Methylresorcin | 0,009 |
| Ammoniak, 25 %ig | ad pH 9,8 |
| Wasser | ad 100 |
| Farbresultat | dämmergrau |

### Rezeptur 2.5

| Rohstoff | Menge in Gew.-% |
|---|---|
| Fettalkoholgemisch C₁₂-C₁₈ | 10,0 |
| Texapon® K14 S70C | 2,5 |
| Plantaren® 1200 UP | 2,0 |
| Akypo Soft® 45 NV | 12,0 |
| Eutanol® G | 1,0 |
| Eumulgin® B 1 | 0,5 |
| Eumulgin® B2 | 0,5 |
| Polymer W 37194 | 2,0 |
| Cosmedia Guar® C261 | 0,2 |
| Mirapol® A15 | 0,5 |
| Ascorbinsäure | 0,4 |
| Ammoniumsulfat | 0,5 |
| E2 (erfindungsgemäß) | 0,92 |
| Bis-(5-amino-2-hydroxyphenyl)methan-dihydrochlorid | 0,124 |
| p-Toluylendiamin-sulfat | 0,59 |
| 4-Amino-2-aminomethylphenol-dihydrochlorid | 0,01 |
| 4-Amino-3-methylphenol | 0,077 |
| 2,4,5,6-Tetraaminopyrimidin-sulfat | 0,08 |
| 4-Hydroxy-2,5,6-triaminopyrimidin-sulfat | 0,02 |
| 4,5-Diamino-1-(2'-Hydroxyethyl) pyrazol-sulfat | 0,02 |
| 2,7-Dihydroxynaphthalin | 0,035 |
| 2-Amino-3-hydroxypyridin | 0,44 |
| 3-Amino-2-methylamino-6-methoxypyridin | 0,002 |
| 2,6-Dihydroxy-3,4-dimethylpyridin | 0,002 |
| Resorcin | 0,12 |
| 2-Methylresorcin | 0,72 |
| 3-Aminophenol | 0,007 |
| 4-Amino-2-nitro-diphenylamin-2'-carbonsäure | 0,05 |
| 2-Ethylamino-4-nitro-6-chlorphenol | 0,05 |
| Ammoniak, 25 %ig | ad pH 9,8 |
| Wasser | ad 100 |
| Farbresultat | dunkelrubin |

### Rezeptur 2.6

| Rohstoff | Menge in Gew.-% |
|---|---|
| Fettalkoholgemisch C₁₂-C₁₈ | 10,0 |
| Texapon® K14 S70C | 2,5 |
| Plantaren® 1200 UP | 2,0 |
| Akypo Soft® 45 NV | 12,0 |
| Eutanol® G | 1,0 |
| Eumulgin® B1 | 0,5 |
| Eumulgin® B2 | 0,5 |
| Polymer W 37194 (Stockhausen) | 2,0 |
| Cosmedia Guar® C261 | 0,2 |
| Mirapol® A15 | 0,5 |
| Ascorbinsäure | 0,4 |
| Ammoniumsulfat | 0,5 |
| E3 (erfindungsgemäß) | 0,03 |
| Bis-(5-amino-2-hydroxyphenyl)methan-dihydrochlorid | 2,40 |
| 4-Amino-2-((diethylamino) methyl) phenol-dihydrochlorid | 0,18 |
| 3-Amino-2-methylamino-6-methoxypyridin | 2,04 |
| 2,6-Dihydroxy-3,4-dimethylpyridin | 0,01 |
| 3,5-Diamino-2,6-dimethoxypyridin | 0,02 |
| 2-Ethylamino-4-nitro-6-chlorphenol | 0,07 |
| HC Red BN¹⁸ | 0,2 |
| 5,6-Dihydroxyindolin-hydrobromid | 0,12 |
| Ammoniak, 25 %ig | ad pH 9,8 |
| Wasser | ad 100 |
| Farbresultat | hennarot |

### Rezeptur 2.7

| Rohstoff | Menge in Gew.-% |
|---|---|
| Fettalkoholgemisch C₁₂-C₁₈ | 8,0 |
| Texapon® NSO | 2,0 |
| Dehyton® K | 1,0 |
| Kaliumoleat | 2,0 |
| Kaliumisostearat | 2,0 |
| Myristinsäure | 1,0 |
| Eumulgin® B2 | 0,5 |
| Custofac Diacid® H 240¹⁹ | 2,0 |
| Merquat® 550²⁰ | 0,2 |
| Luviquat® FC 370²¹ | 0,1 |
| Merquat® 280²² | 0,1 |
| Gafquat® HS-100²³ | 0,1 |
| Ascorbinsäure | 0,4 |
| Ammoniumsulfat | 0,5 |
| E1 (erfindungsgemäß) | 0,05 |
| Bis-(5-amino-2-hydroxyphenyl)methan-dihydrochlorid | 2,33 |
| 5-Amino-2-methylphenol | 0,6 |
| 5-(2'-Hydroxyethyl) amino-2-methylphenol | 0,3 |
| 3-Amino-2-chlor-6-methylphenol | 0,5 |
| Ammoniak, 25 %ig | ad pH 9,8 |
| Wasser | ad 100 |
| Farbresultat | rotgold |

### Rezeptur 2.8

| Rohstoff | Menge in Gew.-% |
|---|---|
| Fettalkoholgemisch C₁₂-C₁₈ | 8,0 |
| Texapon® NSO | 2,0 |
| Dehyton® K | 1,0 |
| Kaliumoleat | 2,0 |
| Kaliumisostearat | 2,0 |
| Myristinsäure | 1,0 |
| Eumulgin® B2 | 0,5 |
| Custofac Diacid® H 240 | 2,0 |
| Merquat® 550 | 0,2 |
| Luviquat® FC 370 | 0,1 |
| Merquat® 280 | 0,1 |
| Gafquat® HS-100 | 0,1 |
| Ascorbinsäure | 0,4 |
| Ammoniumsulfat | 0,5 |
| E2 (erfindungsgemäß) | 0,37 |
| Bis-(5-amino-2-hydroxyphenyl)methan-dihydrochlorid | 1,24 |
| p-Toluylendiamin-sulfat | 0,15 |
| 2-(2'-Hydroxyethyl)-p-phenylendiamin-sulfat | 0,10 |
| 2-Amino-4-methylphenol | 0,01 |
| 4,5-Diamino-1-(2'-hydroxyethylpyrazol)-sulfat | 0,24 |
| 2,7-Dihydroxynaphthalin | 0,10 |
| 5-Amino-2-methylphenol | 0,15 |
| 5-(2'-Hydroxyethyl) amino-2-methylphenol | 0,44 |
| 5-Amino-4-chlor-2-methylphenol | 0,1 |
| Resorcin | 0,04 |
| 2-Methylresorcin | 0,08 |
| 4-Chlorresorcin | 0,05 |
| Ammoniak, 25 %ig | ad pH 9,8 |
| Wasser | ad 100 |
| Farbresultat | maron |

### Rezeptur 2.9

| Rohstoff | Menge in Gew.-% |
|---|---|
| Fettalkoholgemisch C₁₂-C₁₈ | 8,0 |
| Texapon® NSO | 2,0 |
| Dehyton® K | 1,0 |
| Kaliumoleat | 2,0 |
| Kaliumisostearat | 2,0 |
| Myristinsäure | 1,0 |
| Eumulgin® B2 | 0,5 |
| Custofac Diacid® H 240 | 2,0 |
| Merquat® 550 | 0,2 |
| Luviquat® FC 370 | 0,1 |
| Merquat® 280 | 0,1 |
| Gafquat® HS-100 | 0,1 |
| Ascorbinsäure | 0,4 |
| Ammoniumsulfat | 0,5 |
| E3 (erfindungsgemäß) | 0,09 |
| Bis-(5-amino-2-hydroxyphenyl)methan-dihydrochlorid | 0,038 |
| p-Phenylendiamin-dihydrochlorid | 0,10 |
| 4-Amino-2-aminomethylphenol-dihydrochlorid | 0,3 |
| 4-Amino-2-((diethylamino) methyl) phenol-dihydrochlorid | 0,8 |
| 4-Amino-3-methylphenol | 0,011 |
| 2,4,5,6-Tetraaminopyrimidin-sulfat | 0,80 |
| 4-Hydroxy-3,5,6-triaminopyrimidinsulfat | 0,08 |
| 2,7-Dihydroxynaphthalin | 0,33 |
| 5-(2'-Hydroxyethyl) amino-2-methylphenol | 0,015 |
| 5-Amino-4-chlor-2-methylphenol | 0,030 |
| Resorcin | 0,044 |
| 2-Methylresorcin | 0,25 |
| 3-Aminophenol | 0,004 |
| 4-Amino-2-nitro-diphenylamin-2'-carbonsäure | 0,15 |
| 4-Amino 3-nitrophenol | 0,25 |
| Ammoniak, 25%ig | ad pH 9,8 |
| Wasser | ad 100 |
| Farbresultat | fuchsrot |

### Rezeptur 2.10

| Rohstoff | Menge in Gew.-% |
|---|---|
| Fettalkoholgemisch C₁₂-C₁₈ | 8,0 |
| Texapon® NSO | 2,0 |
| Dehyton® K | 1,0 |
| Kaliumoleat | 2,0 |
| Kaliumisostearat | 2,0 |
| Myristinsäure | 1,0 |
| Eumulgin® B2 | 0,5 |
| Custofac Diacid® H 240 | 2,0 |
| Merquat® 550 | 0,2 |
| Luviquat® FC 370 | 0,1 |
| Merquat® 280 | 0,1 |
| Gafquat® HS-100 | 0,1 |
| Ascorbinsäure | 0,4 |
| Ammoniumsulfat | 0,5 |
| E1 (erfindungsgemäß) | 0,02 |
| E2 (erfindungsgemäß) | 0,02 |
| E3 (erfindungsgemäß) | 0,02 |
| Bis-(5-amino-2-hydroxyphenyl)methan-dihydrochlorid | 1,47 |
| 4-Amino-3-methylphenol | 0,1 |
| 3-Amino-2-methylamino-6-methoxypyridin | 0,05 |
| 2,6-Dihydroxy-3,4-dimethylpyridin | 0,01 |
| 3,5-Diamino-2,6-dimethoxypyridin | 0,05 |
| 5-Amino-2-methylphenol | 0,40 |
| 5-(2'-Hydroxyethyl) amino-2-methylphenol | 0,30 |
| 3-Amino-2-chlor-6-methylphenol | 0,06 |
| 3-Amino-2,4-dichlorphenol | 0,04 |
| 2,4-Diaminophenoxyethanol-sulfat | 0,01 |
| 1,3-Bis-(2',4'-diaminophenoxy) propan-tetrahydrochlorid | 0,1 |
| 1,3-Bis-(2',4'-diamiphenyl) propan-tetrahydrochlorid | 0,2 |
| 4-Hydroxyindol | 0,1 |
| Ammoniak, 25 %ig | ad pH 9,8 |
| Wasser | ad 100 |
| Farbresultat | Somali |

### Rezeptur 2.11

| Rohstoff | Menge in Gew.-% |
|---|---|
| Fettalkoholgemisch C₁₂-C₁₈ | 10,0 |
| Eumulgin® B1 | 1,0 |
| Eumulgin® B2 | 1,0 |
| Texapon® NSO | 3,0 |
| Dehyton® K | 1,0 |
| Akypo® RLM 45 N²⁴ | 3,0 |
| Aminoxid® WS 35²⁵ | 0,5 |
| Merquat® 100²⁶ | 0,05 |
| Merquat® 280 | 0,05 |
| Polymer JR® 400 | 0,05 |
| Mirapol® A 15 | 0,05 |
| Silkall® 100²⁷ | 0,5 |
| Ammoniumsulfat | 0,4 |
| Turpinal® SL²⁸ | 0,2 |
| Nutrilan Keratin® W²⁹ | 1,0 |
| Natronwasserglas 40/42 | 0,5 |
| E1 (erfindungsgemäß) | 0,08 |
| 2,4,5,6-Tetraaminopyrimidin-sulfat | 1,0 |
| 2,6-Bis-(2'-hydroxyethylamino)-toluol | 0,08 |
| p-Toluylendiamin-sulfat | 0,10 |
| Parfüm | 0,2 |
| 2-Methylresorcin | 0,59 |
| HC Red B 54³⁰ | 0,05 |
| HC Red BN | 0,05 |
| 1,4-Diamino-2-nitrobenzol | 0,2 |
| Acid Red 52 | 0,05 |
| Ammoniak, konz | ad pH 10 |
| Wasser | ad 100 |
| Farbresultat | erdbeerrot |

### Rezeptur 2.12

| Rohstoff | Menge in Gew.-% |
|---|---|
| Fettalkoholgemisch C₁₂-C₁₈ | 10,0 |
| Eumulgin® B1 | 1,0 |
| Eumulgin® B2 | 1,0 |
| Texapon® NSO | 3,0 |
| Dehyton® K | 1,0 |
| Akypo RLM® 45 N | 3,0 |
| Aminoxid® WS 35 | 0,5 |
| Merquat® 100 | 0,05 |
| Merquat® 280 | 0,05 |
| Polymer JR® 400 | 0,05 |
| Mirapol® A 15 | 0,05 |
| Silkall® 100 | 0,5 |
| Ammoniumsulfat | 0,4 |
| Turpinal® SL | 0,2 |
| Nutrilan Keratin® W | 1,0 |
| Natronwasserglas 40/42 | 0,5 |
| Parfüm | 0,2 |
| E2 (erfindungsgemäß) | 0,30 |
| p-Toluylendiamin-sulfat | 0,22 |
| 5-Amino-2-methylphenol | 0,25 |
| 3-Amino-2-chlor-6-methylphenol | 0,07 |
| Acid Red 33 | 0,05 |
| Wasser | ad 100 |
| Ammoniak, konz | ad pH 10 |
| Farbresultat | magenta |

### Rezeptur 2.13

| Rohstoff | Menge in Gew.-% |
|---|---|
| Fettalkoholgemisch C₁₂-C₁₈ | 10,0 |
| Eumutgin® B1 | 1,0 |
| Eumulgin® B2 | 1,0 |
| Texapon® NSO | 3,0 |
| Dehyton® K | 1,0 |
| Akypo RLM® 45 N | 3,0 |
| Aminoxid WS® 35 | 0,5 |
| Merquat® 100 | 0,05 |
| Merquat® 280 | 0,05 |
| Polymer JR® 400 | 0,05 |
| Mirapol® A 15 | 0,05 |
| Silkall® 100 | 0,5 |
| Ammoniumsulfat | 0,4 |
| Turpinal® SL | 0,2 |
| Nutrilan Keratin® W | 1,0 |
| Natronwasserglas 40/42 | 0,5 |
| Parfüm | 0,2 |
| E3 (erfindungsgemäß) | 0,31 |
| p-Toluylendiamin-sulfat | 0,16 |
| 4-Amino-3-methylphenol | 0,43 |
| 2,7-Dihydroxynaphthalin | 0,02 |
| Resorcin | 0,17 |
| 5-Amino-2-methylphenol | 0,14 |
| 4-Amino-2-nitro-diphenylamin-2'-carbonsäure | 0,05 |
| 6-Nitro-1,2,3,4-tetrahydrochinoxalin | 0,03 |
| Rodol® 9R³¹ | 0,02 |
| Ammoniak, konz. | ad pH 10 |
| Wasser | ad 100 |
| Farbresultat | rehbraun |

### Rezeptur 2.14

| Rohstoff | Menge in Gew.-% |
|---|---|
| Dipicolinsäure | 0,10 |
| Natriumpyrophosphat | 0,03 |
| Turpinal® SL | 1,50 |
| Texapon® N28³² | 2,00 |
| Dow Corning® DB 110A³³ | 0,07 |
| Aculyn® 33³⁴ | 12,00 |
| Wasserstoffperoxid, 50%ig | 12,00 |
| Ammoniak, 25%ig | 0,60 |
| Wasser | ad 100 |

Die im Rahmen der Beispiele eingesetzten Handelsprodukte haben die folgenden Inhaltsstoffe:
7 quaternierte Hydroxyethylcellulose (INCI-Bezeichnung: Polyquaternium-10) (Amerchol)
8 quaterniertes Vinylpyrrolidon-Dimethylaminoethylmethacrylat-Copolymer (ca. 19-21 % Aktivsubstanzgehalt; INCI-Bezeichnung: Polyquaternium-11) (ISP)
9 quaterniertes Cellulose-Derivat (INCI-Bezeichnung: Polyquaternium-4) (National Starch)
10 Laurylmyristylethersulfat-Natrium-Salz (ca. 68 bis 73% Aktivsubstanzgehalt; INCI-Bezeichnung: Sodium Myreth Sulfate) (Cognis)
11 C₁₂₋₁₆-Fettalkohol-1.4-glucosid (ca. 50 - 53% Aktivsubstanzgehalt; INCI-Bezeichnung: Lauryl Glucoside, Aqua (Water)) (Cognis)
12 C₁₂₋₁₄-Fettalkohol-4.5-EO-Essigsäure-Natrium-Salz (mind. 21% Aktivsubstanzgehalt; INCI-Bezeichnung: Sodium Laureth-6 Carboxylate) (Kao)
13 2-Octyldodecylalkohol (INCI-Bezeichnung: Octyldodecanol) (Cognis)
14 Cetylstearylalkohol mit ca. 12-EO-Einheiten (INCI-Bezeichnung: Ceteareth-12) (Cognis)
15 Acrylsäure-Natrium-Salz-Acrylamidopropyltrimethylammoniumchlorid-Copolymer konserviert mit Phenonip (ca. 19-21% Aktivsubstanz, INCI-Bezeichnung: Acrylamidopropyl-Trimonium Chloride / Acrylates Copolymer) (Stockhausen)
16 Guarhydroxypropyltrimethylammoniumchlorid (INCI-Bezeichnung: Guar Hydroxypropyltrimonium Chloride) (Cognis)
17 Poly[N-(3-(dimethylammonium)propyl]-N'-[3-ethylenoxyethylen- dimethylammonium)propyl]-harnstoff-di-chlorid (ca. 64% Aktivsubstanzgehalt; INCI-Bezeichnung: Polyquatemium-2) (Rhodia)
18 4[(3-Hydroxypropyl)amino]-3-nitrophenol
19 4-Hexyl-5(6)-carboxy-2-cyclohexen-1-octansäure-Kalium-Salz (ca. 41% Aktivsubstanzgehalt) (Westvaco Chemicals)
20 Dimethyldiallylammoniumchlorid-Acrylamid-Copolymer (ca. 8,1 - 9,1 % Aktivsubstanzgehalt; INCI-Bezeichnung: Polyquaternium-7) (Ondeo-Nalco)
21 Vinylimidazoliummethochlorid-Vinylpyrrolidon-Copolymerisat (30:70) (ca. 38 - 42% Aktivsubstanzgehalt; INCI-Bezeichnung: Polyquaternium-16) (BASF)
22 Dimethyldiallylammoniumchlorid-Acrylsäure-Copolymer (ca. 35% Aktivsubstanz in Wasser, INCI-Bezeichnung: Polyquaternium-22) (Ondeo-Nalco)
23 Vinylpyrrolidon-Methacrylamidopropyltrimethylammoniumchlorid-Copolymer (ca. 19 - 21 % Aktivsubstanzgehalt in Wasser; INCI-Bezeichnung: Polyquaternium-28) (Gaf Corp.)
24 C₁₂₋₁₄-Fettalkohol-4.5-EO-Essigsäure-Natrium-Salz (ca. 80-84% Aktivsubstanzgehalt in Wasser und NaCl; INCI-Bezeichnung: Sodium Laureth-6 Carboxylate) (Kao)
25 N,N-Dimethyl-N(C₈₋₁₈-kokosacylamidopropyl)amin-N-oxid (ca. 35% Aktivsubstanzgehalt in Wasser; INCI-Bezeichnung: Cocamidopropylamine Oxide) (Goldschmidt)
26 Poly(dimethyldiallylammoniumchlorid) (ca. 40% Aktivsubstanzgehalt; INCI-Bezeichnung: Polyquaternium-6) (Ondeo-Nalco)
27 Seidenprotein (INCI-Bezeichnung: Silk Serica (Linne)) (Ikeda Bussan Kaisha)
28 1-Hydroxyethan-1,1-diphosphonsäure (ca. 58-61% Aktivsubstanzgehalt in Wasser; INCI-Bezeichnung: Etidronic Acid, Aqua (Water)) (Solutia)
29 enzymatisch gewonnenes Hydrolysat aus Merinoschurwolle (ca. 21-23% Aktivsubstanzgehalt; INCI-Bezeichnung: Aqua (Water), Hydrolyzed Keratin, Phenoxyethanol, Methylparaben, Butylparaben, Ethylparaben, Propylparaben) (Cognis)
30 4-[(2-Hydroxyethyl)amino]-3-nitrophenol (INCI-Bezeichnung: 3-Nitro-p-hydroxyethylaminophenol)
31 2-Amino-6-chloro-4-nitrophenol
32 Laurylethersulfat-Natrium-Salz (mind. 26,5 % Aktivsubstanzgehalt; INCI-Bezeichnung: Sodium Laureth Sulfate) (Cognis)
33 Nichtionogene Silicon Emulsion (10% Aktivsubstanzgehalt; INCI-Bezeichnung: Dimethicone) (Dow Corning)
34 Acrylpolymer (ca. 28 % Aktivsubstanzgehalt; INCI-Bezeichnung: Acrylates Copolymer) (Rohm % Haas)

## Patentansprüche

1. Mittel zum Färben keratinischer Fasern, insbesondere menschlicher Haare, **dadurch gekennzeichnet, dass** es in einem kosmetisch akzeptablen Träger mindestens ein p-Phenylendiaminderivat der Formel (I) 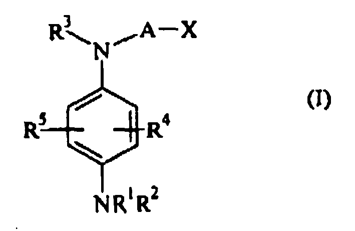 enthält, wobei
A steht für eine verzweigte oder unverzweigte Alkylengruppe mit 1 bis 6 Kohlenstoffatomen, die gegebenenfalls einen oder mehrere Substituenten, ausgewählt aus Hydroxygruppe(n) und Halogenatom(en), tragen kann,
X steht für einen gegebenenfalls substituierten Imidazolylrest,
R¹, R² und R³ stehen unabhängig voneinander für ein Wasserstoffatom, eine C₁- bis C₄-Alkylgruppe, eine C₁- bis C₄-Monohydroxyalkylgruppe oder eine C₂- bis C₆-Polyhydroxyalkylgruppe, und
R⁴ und R⁵ stehen unabhängig voneinander für ein Wasserstoffatom, eine C₁- bis C₄-Alkylgruppe, eine C₁- bis C₄-Alkoxygruppe, eine C₁- bis C₄-Monohydroxyalkylgruppe, eine C₂- bis C₄- Polyhydroxyalkylgruppe oder ein Halogenatom.

2. Mittel nach Anspruch 1, **dadurch gekennzeichnet, dass** R¹, R² und R³ stehen für ein Wasserstoffatom

3. Mittel nach einem der Ansprüche 1 oder 2, **dadurch gekennzeichnet, dass** R⁴ und R⁵ stehen unabhängig voneinander für ein Wasserstoffatom, eine Methylgruppe, ein Chlor- oder ein Fluoratom.

4. Mittel nach Anspruch 3, **dadurch gekennzeichnet, dass** R⁴ und R⁵ für ein Wasserstoffatom stehen.

5. Mittel nach einem der Ansprüche 1 bis 4, **dadurch gekennzeichnet, dass** die Gruppe X steht für einen gegebenenfalls substituierten Imidazolylrest der Formel (II) 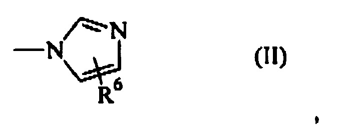 wobei R⁶ steht für ein Wasserstoffatom, eine C₁- bis C₄-Alkylgruppe, eine C₁- bis C₄-Monohydroxyalkylgruppe, eine C₂- bis C₄- Polyhydroxyalkylgruppe oder ein Halogenatom.

6. Mittel nach Anspruch 5, **dadurch gekennzeichnet, dass** A steht für eine unverzweigte Alkylengruppe mit 2 bis 6 Kohlenstoffatomen.

7. Mittel nach Anspruch 6, **dadurch gekennzeichnet, dass** A steht für eine Trimethylengruppe.

8. Mittel nach einem der Ansprüche 5 bis 7, **dadurch gekennzeichnet, dass** die Verbindung der Formel (I) (4-Aminophenyl)(3-(imidazol-1-yl)propyl)amin ist.

9. Mittel nach einem der Ansprüche 1 bis 4, **dadurch gekennzeichnet, dass** die Gruppe X steht für einen gegebenenfalls substituierten Imidazolylrest der Formel (III) 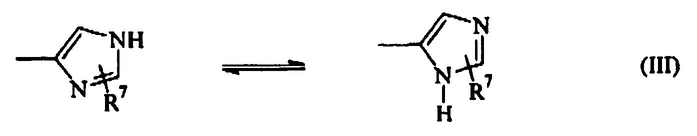 wobei R⁷ steht für ein Wasserstoffatom, eine C₁- bis C₄-Alkylgruppe, eine C₁- bis C₄-Monohydroxyalkylgruppe, eine C₂- bis C₄- Polyhydroxyalkylgruppe oder ein Halogenatom.

10. Mittel nach Anspruch 9, **dadurch gekennzeichnet, dass** A steht für eine unverzweigte Alkylengruppe mit 1 bis 6 Kohlenstoffatomen.

11. Mittel nach Anspruch 10, **dadurch gekennzeichnet, dass** A steht für eine Ethylengruppe.

12. Mittel nach einem der Ansprüche 9 bis 11, **dadurch gekennzeichnet, dass** die Verbindung der Formel (I) (4-Aminophenyl)(2-(imidazol-5-yl)ethyl)amin ist.

13. Verwendung von Mitteln nach einem der Ansprüche 1 bis 12 zum Färben keratinischer Fasern.

14. Verfahren zur Färbung keratinischer Fasern, **dadurch gekennzeichnet, dass** ein Mittel nach einem der Ansprüche 1 bis 12 auf die Fasern aufgetragen wird und nach einer Einwirkzeit wieder abgespült wird.

15. p-Phenylendiaminderivate der Formel (I) 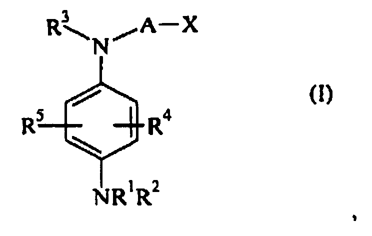 **dadurch gekennzeichnet, dass**
A steht für eine verzweigte oder unverzweigte Alkylengruppe mit 1 bis 6 Kohlenstoffatomen, die einen oder mehrere Substituenten, ausgewählt aus Hydroxygruppe(n) und Halogenatom(en), tragen kann,
X steht für einen gegebenenfalls substituierten Imidazolylrest der Formel (III), 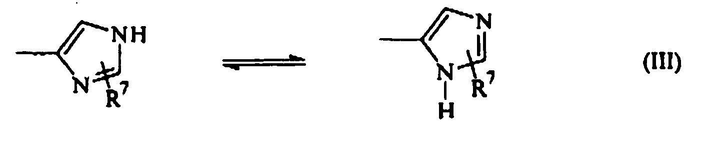
R¹, R² und R³ stehen unabhängig voneinander für ein Wasserstoffatom, eine C₁- bis C₄-Alkylgruppe, eine C₁- bis C₄-Monohydroxyalkylgruppe oder eine C₂- bis C₆-Polyhydroxyalkylgruppe,
R⁴ und R⁵ stehen unabhängig voneinander für ein Wasserstoffatom, eine C₁- bis C₄-Alkylgruppe, eine C₁- bis C₄-Alkoxygruppe, eine C₁- bis C₄-Monohydroxyalkylgruppe, eine C₂- bis C₄- Polyhydroxyalkylgruppe oder ein Halogenatom und
R⁷ steht für ein Wasserstoffatom, eine C₁- bis C₄-Alkylgruppe, eine C₁- bis C₄-Monohydroxyalkylgruppe, eine C₂- bis C₄- Polyhydroxyalkylgruppe oder ein Halogenatom.

16. p-Phenylendiaminderivate gemäß Anspruch 15, **dadurch gekennzeichnet, dass** es (4-Aminophenyl)(2-(imidazol-5-yl)ethyl)amin ist.

## Claims

1. Composition for dyeing keratinic fibers, in particular human hair, **characterized in that** it contains, in a cosmetically acceptable carrier, at least one p-phenylenediamine derivative of the formula (I) 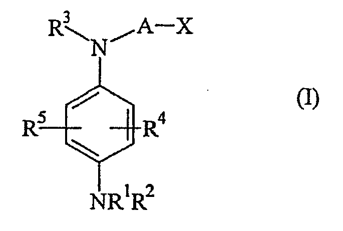 where
A is a branched or unbranched alkylene group having 1 to 6 carbon atoms, which can optionally carry one or more substituents, selected from hydroxy group(s) and halogen atom(s),
X is an optionally substituted imidazolyl radical,
R¹, R² and R³ are, independently of one another, a hydrogen atom, a C₁- to C₄-alkyl group, a C₁- to C₄-monohydroxyalkyl group or a C₂- to C₆-polyhydroxyalkyl group, and
R⁴ and R⁵ are, independently of one another, a hydrogen atom, a C₁- to C₄-alkyl group, a C₁- to C₄-alkoxy group, a C₁- to C₄-monohydroxyalkyl group, a C₂- to C₄-polyhydroxyalkyl group or a halogen atom.

2. Composition according to Claim 1, **characterized in that** R¹, R² and R³ are a hydrogen atom.

3. Composition according to one of Claims 1 or 2, **characterized in that** R⁴ and R⁵ are, independently of one another, a hydrogen atom, a methyl group, a chlorine or a fluorine atom.

4. Composition according to Claim 3, **characterized in that** R⁴ and R⁵ are a hydrogen atom.

5. Composition according to one of Claims 1 to 4, **characterized in that** the group X is an optionally substituted imidazolyl radical of the formula (II) 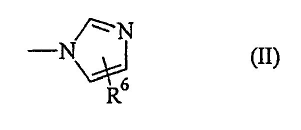 where R⁶ is a hydrogen atom, a C₁ to C₄-alkyl group, a C₁- to C₄-monohydroxyalkyl group, a C₂- to C₄-polyhydroxyalkyl group or a halogen atom.

6. Composition according to Claim 5, **characterized in that** A is an unbranched alkylene group having 2 to 6 carbon atoms.

7. Composition according to Claim 6, **characterized in that** A is a trimethylene group.

8. Composition according to one of Claims 5 to 7, **characterized in that** the compound of the formula (I) is (4-aminophenyl)(3-(imidazol-1-yl)propyl)-amine.

9. Composition according to one of Claims 1 to 4, **characterized in that** the group X is an optionally substituted imidazolyl radical of the formula (III) 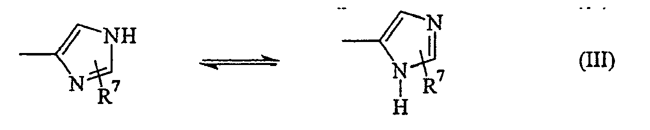 where R⁷ is a hydrogen atom, a C₁- to C₄-alkyl group, a C₁- to C₄-monohydroxyalkyl group, a C₂- to C₄-polyhydroxyalkyl group or a halogen atom.

10. Composition according to Claim 9, **characterized in that** A is an unbranched alkylene group having 1 to 6 carbon atoms.

11. Composition according to Claim 10, **characterized in that** A is an ethylene group.

12. Composition according to one of Claims 9 to 11, **characterized in that** the compound of the formula (I) is (4-aminophenyl)(2-(imidazol-5-yl)ethyl)amine.

13. Use of compositions according to one of Claims 1 to 12 for dyeing keratinic fibers.

14. Process for dyeing keratinic fibers, **characterized in that** a composition according to one of Claims 1 to 12 is applied to the fibers and after a time of action is rinsed off again.

15. p-Phenylenediamine derivatives of the formula (I) 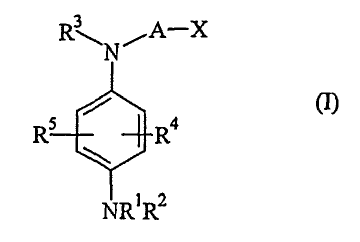 **characterized in that**
A is a branched or unbranched alkylene group having 1 to 6 carbon atoms, which can carry one or more substituents, selected from hydroxy group(s) and halogen atom(s),
X is an optionally substituted imidazolyl radical of the formula (III), 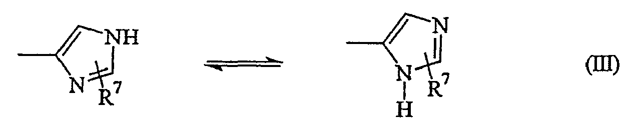
R¹, R² and R³ are, independently of one another, a hydrogen atom, a C₁- to C₄-alkyl group, a C₁- to C₄-monohydroxyalkyl group or a C₂- to C₆-polyhydroxyalkyl group,
R⁴ and R⁵ are, independently of one another, a hydrogen atom, a C₁- to C₄-alkyl group, a C₁- to C₄-alkoxy group, a C₁- to C₄-monohydroxyalkyl group, a C₂- to C₄-polyhydroxyalkyl group or a halogen atom and
R⁷ is a hydrogen atom, a C₁- to C₄-alkyl group, a C₁- to C₄-monohydroxyalkyl group, a C₂- to C₄-polyhydroxyalkyl group or a halogen atom.

16. p-Phenylenediamine derivatives according to Claim 15, **characterized in that** it is (4-aminophenyl) (2-(imidazol-5-yl)ethyl)amine.

## Revendications

1. Agent pour la teinture de fibres kératiniques, en particulier les cheveux humains, **caractérisé en ce qu'**il contient, dans un support cosmétiquement acceptable, au moins un dérivé de la p-phénylènediamine de formule (I) 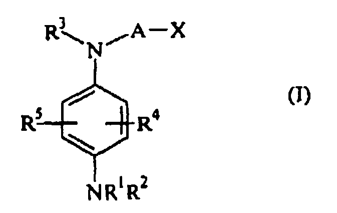 où
A représente un groupe alkylène ramifié ou non ramifié comprenant 1 à 6 atomes de carbone, qui peut le cas échéant porter un ou plusieurs substituants, choisis parmi les groupes hydroxy et les atomes d'halogène,
X représente un radical imidazolyle le cas échéant substitué,
R¹, R² et R³ représentent, indépendamment l'un de l'autre, un atome d'hydrogène, un groupe alkyle en C₁ à C₄, un groupe monohydroxyalkyle en C₁ à C₄ ou un groupe polyhydroxyalkyle en C₂ à C₆, et
R⁴ et R⁵ représentent, indépendamment l'un de l'autre, un atome d'hydrogène, un groupe alkyle en C₁ à C₄, un groupe alcoxy en C₁ à C₄, un groupe monohydroxyalkyle en C₁ à C₄, un groupe polyhydroxyalkyle en C₂ à C₄ ou un atome d'halogène.

2. Agent selon la revendication 1, **caractérisé en ce que** R¹, R² et R³ représentent un atome d'hydrogène.

3. Agent selon l'une quelconque des revendications 1 ou 2, **caractérisé en ce que** R⁴ et R⁵ représentent, indépendamment l'un de l'autre, un atome d'hydrogène, un groupe méthyle, un atome de chlore ou de fluor.

4. Agent selon la revendication 3, **caractérisé en ce que** R⁴ et R⁵ représentent un atome d'hydrogène.

5. Agent selon l'une quelconque des revendications 1 à 4, **caractérisé en ce que** le groupe X représente un radical imidazolyle le cas échéant substitué de formule (II) 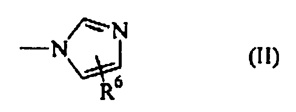 où R⁶ représente un atome d'hydrogène, un groupe alkyle en C₁ à C₄, un groupe monohydroxyalkyle en C₁ à C₄, un groupe polyhydroxyalkyle en C₂ à C₄ ou un atome d'halogène.

6. Agent selon la revendication 5, **caractérisé en ce que** A représente un groupe alkylène non ramifié comprenant 2 à 6 atomes de carbone.

7. Agent selon la revendication 6, **caractérisé en ce que** A représente un groupe triméthylène.

8. Agent selon l'une quelconque des revendications 5 à 7, **caractérisé en ce que** le composé de formule (I) est la (4-aminophényl)(3-(imidazol-1-yl)propyl)amine.

9. Agent selon l'une quelconque des revendications 1 à 4, **caractérisé en ce que** le groupe X représente un radical imidazolyle le cas échéant substitué de formule (III) 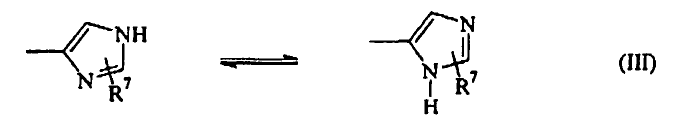 où R⁷ représente un atome d'hydrogène, un groupe alkyle en C₁ à C₄, un groupe monohydroxyalkyle en C₁ à C₄, un groupe polyhydroxyalkyle en C₂ à C₄ ou un atome d'halogène.

10. Agent selon la revendication 9, **caractérisé en ce que** A représente un groupe alkylène non ramifié comprenant 1 à 6 atomes de carbone.

11. Agent selon la revendication 10, **caractérisé en ce que** A représente un groupe éthylène.

12. Agent selon l'une quelconque des revendications 9 à 11, **caractérisé en ce que** le composé de formule (I) est la (4-aminophényl)(2-(imidazol-5-yl)éthyl)amine.

13. Utilisation d'agents selon l'une quelconque des revendications 1 à 12 pour la teinture de fibres kératiniques.

14. Procédé pour la teinture de fibres kératiniques, **caractérisé en ce qu'**on applique un agent selon l'une quelconque des revendications 1 à 12 sur les fibres et on l'élimine à nouveau par rinçage après un temps d'action.

15. Dérivés de p-phénylènediamine de formule (I) 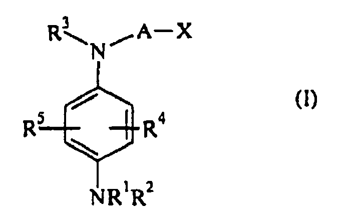 **caractérisés en ce que**
A représente un groupe alkylène ramifié ou non ramifié comprenant 1 à 6 atomes de carbone, qui peut porter un ou plusieurs substituants, choisis parmi les groupes hydroxy et les atomes d'halogène,
X représente un radical imidazolyle le cas échéant substitué de formule (III), 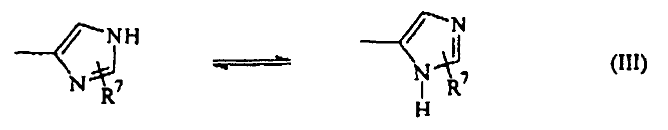
R¹, R² et R³ représentent, indépendamment l'un de l'autre, un atome d'hydrogène, un groupe alkyle en C₁ à C₄, un groupe monohydroxyalkyle en C₁ à C₄ ou un groupe polyhydroxyalkyle en C₂ à C₆,
R⁴ et R⁵ représentent, indépendamment l'un de l'autre, un atome d'hydrogène, un groupe alkyle en C₁ à C₄, un groupe alcoxy en C₁ à C₄, un groupe monohydroxyalkyle en C₁ à C₄, un groupe polyhydroxyalkyle en C₂ à C₄ ou un atome d'halogène et
R⁷ représente un atome d'hydrogène, un groupe alkyle en C₁ à C₄, un groupe monohydroxyalkyle en C₁ à C₄, un groupe polyhydroxyalkyle en C₂ à C₄ ou un atome d'halogène.

16. Dérivés de p-phénylènediamine selon la revendication 15, **caractérisés en ce qu'**il s'agit de la (4-aminophényl)(2-(imidazol-5-yl)éthyl)amine.
